(19) **Europäisches Patentamt / European Patent Office / Office européen des brevets**

(11) **EP 1 933 701 B1**

(12) **EUROPEAN PATENT SPECIFICATION**

(45) Date of publication and mention
of the grant of the patent:
**11.02.2009 Bulletin 2009/07**

(51) Int Cl.:
*A61B 5/027* (2006.01)    *A61B 5/053* (2006.01)

(21) Application number: **06783918.3**

(86) International application number:
**PCT/NL2006/000452**

(22) Date of filing: **12.09.2006**

(87) International publication number:
**WO 2007/032665 (22.03.2007 Gazette 2007/12)**

(54) **METHOD AND DEVICE FOR DETERMINING FLOW IN A BLOOD VESSEL**

VERFAHREN UND VORRICHTUNG ZUR BESTIMMUNG DES DURCHFLUSSES IN EINEM
BLUTGEFÄSS

PROCEDE ET DISPOSITIF PERMETTANT DE DETERMINER L'ECOULEMENT DANS UN
VAISSEAU SANGUIN

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **15.09.2005 NL 1029969
03.08.2006 NL 1032272**

(43) Date of publication of application:
**25.06.2008 Bulletin 2008/26**

(73) Proprietor: **Martil Instruments B.V.
1852 GC Heiloo (NL)**

(72) Inventor: **POP, Gheorghe, Aurel, Marie
6525 ZR Nijmegen (NL)**

(74) Representative: **van Kooij, Adriaan et al
Arnold & Siedsma
Sweelickplein 1
2517 GK The Hague (NL)**

(56) References cited:
EP-A1- 0 591 642     WO-A-00/74775
WO-A-94/22367     WO-A2-03/022143
US-A- 3 149 627     US-A1- 2001 025 188
US-A1- 2005 177 062

• PEURA R A ET AL: "INFLUENCE OF
ERYTHROCYTE VELOCITY ON IMPEDANCE
PLETHYSMOGRAPHIC MEASUREMENTS"
MEDICAL AND BIOLOGICAL ENGINEERING AND
COMPUTING, SPRINGER, HEILDELBERG, DE,
vol. 16, no. 2, March 1978 (1978-03), pages
147-154, XP008075045 ISSN: 0140-0118

• FUJII M ET AL: "Orientation and deformation of
erythrocytes in flowing blood." ANNALS OF THE
NEW YORK ACADEMY OF SCIENCES 20 APR
1999, vol. 873, 20 April 1999 (1999-04-20), pages
245-261, XP008074974 ISSN: 0077-8923

• FUJII M ET AL: "ORIENTATION AND
DEFORMATION OF ERYTHROCYTES DUE TO
FLOW" ENGINEERING IN MEDICINE AND
BIOLOGY SOCIETY, 1998. PROCEEDINGS OF
THE 20TH ANNUAL INTERNATIONAL
CONFERENCE OF THE IEEE HONG KONG,
CHINA 29 OCT.-1 NOV. 1998, PISCATAWAY, NJ,
USA,IEEE, US, 1998, pages 3024-3027,
XP008074988 ISBN: 0-7803-5164-9

• VISSER K R: "ELECTRIC PROPERTIES OF
FLOWING BLOOD AND IMPEDANCE
CARDIOGRAPHY" ANNALS OF BIOMEDICAL
ENGINEERING, PERGAMON PRESS, OXFORD,
GB, vol. 17, no. 5, 1989, pages 463-473,
XP008074972 ISSN: 0090-6964

• VISSER K R: "ELECTRIC CONDUCTIVITY OF
STATIONARY AND FLOWING HUMAN BLOOD AT
LOW FREQUENCIES" MEDICAL AND
BIOLOGICAL ENGINEERING AND COMPUTING,
SPRINGER, HEILDELBERG, DE, vol. 30, no. 6,
November 1992 (1992-11), pages 636-640,
XP000323942 ISSN: 0140-0118

EP 1 933 701 B1

**Description**

The invention relates to a device for determining flow in a blood vessel.

It is known to determine the flow in a blood vessel by means of Doppler measurements in combination with echography. Another known method is a measurement in the heart by means of thermodilution via a Swan-Ganz catheter.

A system and method for measuring cross-sectional areas and pressure gradients in luminal organs is disclosed in WO 2004/075928 A2.

The known methods are either time-consuming and of taxing for the patient, or rather inaccurate. Therefore a method of the stated type may be provided which is not part of the invention and with which a measurement of the flow in a blood vessel can be made accurately and efficiently.

Measurement of the flow in a blood vessel is achieved by

   a) determining the relation between the average shear rate and the impedance of flowing blood,
   b) measuring the impedance in the blood in a cross-section of the blood vessel,
   c) determining the average shear rate from this relation and the measured impedance,
   d) determining the size of the cross-section of the blood vessel,
   e) selecting a theoretical relative flow distribution over the blood vessel cross-section,
   f) determining the average flow speed on the basis of the shear rate and the relative flow distribution, and
   g) determining the flow volume from the determined average flow speed and the cross-section.

The impedance measured in the blood vessel has an precisely determinable relation to the viscosity of the blood, which depends on the momentary shear rate. At a determined flow distribution over the cross-section of the blood vessel the shear rate distribution is also determined.

When the average shear rate is determined by measuring the impedance at a determined location, for instance centrally in the blood vessel, it is possible when the cross-section is also known to determine the average flow speed, and therefore the flow volume in the blood vessel, on the basis of the flow pattern.

At a constant temperature the viscosity of blood is determined by a number of factors, including the flow volume and more particularly the shear rate. These are important factors since blood is a non-Newtonian liquid, which means that the viscosity thereof varies with different shear rates. At lower shear rates the blood viscosity increases sharply because the red blood cells tend to group together ("rouleaux formation"). At increasing shear rates the rouleaux formation disintegrates and the red blood cells tend to move one behind the other in the direction of flow, wherein the viscosity decreases and finally becomes practically constant.

In addition to the flow conditions, the hematocrit value determines the blood viscosity and thus the impedance. At higher hematocrit values the tendency of the red blood cells to group together increases because more cells are present and the distance between them decreases. At increasing hematocrit values the viscosity thus increases. At a fixed shear rate the hematocrit will determine 90% of the blood viscosity. Another factor which is important is the "glue" between the red blood cells during the grouping which is formed by determined macromolecules, of which fibrinogen is the most important. At a fixed shear rate and hematocrit value the fibrinogen will determine 5% of the viscosity.

The blood viscosity plays an important part in the occurrence of thrombosis and is the most important factor in the microcirculatory blood supply of each organ. The evaluation of the blood viscosity and the measurement thereof is therefore advantageous in the cardiovascular field in preventing thrombosis and embolism, while in intensive care conditions the blood supply to critical organs can be improved and the peripheral resistance reduced. Since an increased grouping together of red blood cells further occurs in the case of an inflammation, it has been found that hyperviscosity is an indicator of inflammatory activity.

In respect of determining the viscosity of blood by or using impedance measurements, it is now possible to measure flow volume using the same impedance measurement, which can for instance be useful in determining the cardiac output of the heart.

A favourable further development of the above method is that the steps a, c, e, and f are approximated by determining the relation between the average flow speed and the impedance of flowing blood, and determining the average flow speed from this relation and the measured impedance. Although the viscosity and the impedance of the blood depend on the shear rate, when the shear rate varies a certain delay occurs in the adjustment of the corresponding variation in the viscosity and impedance. This is caused in that the rouleaux formation and the disintegration thereof requires some time. Due to this delay the viscosity will be quite uniform in a non-laminar flow or in a laminar flow which occurs shortly after a non-laminar flow. The influence of the flow distribution is hereby less significant, and there is a usable relation between the viscosity and the impedance on the one hand and the average flow speed on the other.

Use is made hereof in the above method wherein the steps a, c, e, and f are approximated by determining the relation between the average flow speed and the impedance of flowing blood, and determining the average flow speed from this relation and the measured impedance. The above method becomes simpler by determining and using the relation between the average flow speed and the impedance.

If it is indeed required to determine the cardiac output of the heart, i.e. the amount of blood which the heart can pump per unit of time, the blood vessel is the right atrium of a heart and the determined flow volume is that of the heart.

In order to be able to measure the impedance of the blood in reliable manner the impedance measurement is performed with a catheter introduced into the right atrium.

Since the flow speed, and thus the shear rate, varies during the heart cycle, the impedance measurement is performed in a determined period of the ECG. By always performing the measurement in the same period of the ECG a readily comparable measurement value is obtained.

In order to further improve the quality of the measurement value the impedance measurement is performed during a number of heart cycles, in each case in the determined period of the ECG, and the average of the number of measurements is used to determine the impedance.

Incidental differences in flow speed, and thus in impedance, are hereby equalized over the number of heart cycles.

It has been found that the measurement in the right atrium preferably takes place in a period when the atrium is well-dilated, whereby the interference of the electrical field around the catheter by the wall of the right atrium is low. A suitable period is therefore the end of the systole. The measurement preferably takes place in suitable manner during the diastole. A regular flow then occurs which is readily reproducible.

As noted above, other parameters are also important for the absolute value of the viscosity, and thus of the impedance. For a full determination of the flow speed using the above method these parameters must thus be predetermined. A method herefore is characterized in that determination of the relation between the average shear rate and the impedance in flowing blood further comprises of determining in vitro factors co-determining the impedance of blood, such as the hematocrit and fibrinogen content.

The determination of hematocrit and of the fibrinogen content are generally known measuring methods. They can be carried out independently of the impedance measurement. The values normally vary only gradually. Only in acute situations such as heavy bleeding (hematocrit) or serious infections (fibrinogen) will they vary more rapidly. The measurements can therefore normally be carried out in the blood vessel some time before or after the impedance measurement.

Instead of determining the hematocrit and fibrinogen content individually, it is also a useful possibility to apply the method wherein determination of the relation between the shear rate and the impedance in flowing blood further comprises of determining in vitro the average shear rate at which the impedance measured in the blood vessel occurs, and preferably wherein determination of the relation between the average flow speed and the impedance in flowing blood further comprises of determining in vitro the average flow speed at which the impedance measured in the blood vessel occurs. The specific relation in the relevant blood between the shear rate or the flow speed and the impedance is in fact hereby measured, wherein the influence of the hematocrit and fibrinogen is inherent in the determination.

Another suitable example of the above method is characterized in that steps a, c, f and g are performed by generating a blood flow with the chosen relative flow distribution in a vessel of a determined cross-section, measuring the impedance centrally in this vessel in relation to the flow volume of the blood flow and, from the flow volume corresponding with the impedance measured in the blood vessel in accordance with this relation, determining the flow volume in the blood vessel in accordance with the respective sizes of the cross-sections of the blood vessel and the vessel. The flow in the relevant blood vessel is as it were simulated here, whereby a relation between impedance and flow speed is obtained for actual conditions. Only the scale then has to be taken into account in order to directly determine the flow.

A suitable method for determining the size of the blood vessel cross-section is echography. This type of dimension can hereby be determined with considerable accuracy.

It has been found that by applying the above method wherein as theoretical relative flow distribution over the blood vessel cross-section a relative flow distribution is chosen which a Newtonian liquid flowing in laminar manner would display over such a cross-section, a sufficient accuracy can be achieved for the purpose of determining the flow volume. This is particularly the case in combination with the above method wherein the impedance measurement is performed in a determined period of the ECG.

The invention relates to and provides a device for determining the flow of a blood vessel, as characterized in claim 1. The computing means can herein be embodied such that a flow speed or flow volume value is calculated from the measured impedance value. Other parameters, such as the hematocrit and fibrinogen value, as well as the section or diameter of the blood vessel, must of course be entered into the device first for this purpose.

A further development is characterized in claim 3. With this addition a value can be determined, using which the impedance value can be converted to the flow volume.

The invention will be further elucidated in the following description with reference to the accompanying figures.

Figure 1 shows the electrical model of blood in connection with exciting and measuring electrodes.
Figure 2 shows a diagram of a preferred embodiment of the device according to the invention.
Figure 3 shows in partly schematic view a catheter for use with the device according to the invention.

Figure 4 is a cross-section along line IV in figure 3.

Figure 5 is a view as according to arrow V in figure 3.

Figure 6 shows schematically a device for in vitro determination of blood data essential to the present invention.

Figure 7 shows a graph of measurement results obtained with the device of figure 6.

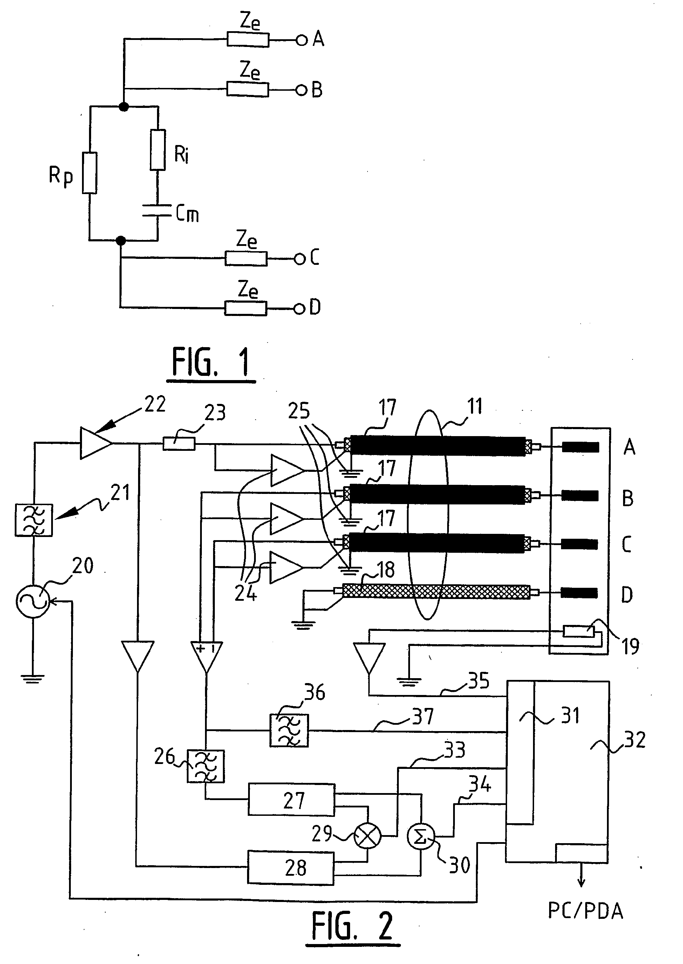

Figure 1 shows the simplified electrical three-element model of blood. An exciting alternating current voltage is generated between electrodes A and D and the measurement is performed between electrodes B and C.

The simplified electrical model comprises the plasma resistance $R_p$ and the cell membrane capacitance $C_m$. It is known that $C_m$ in particular has a strong correlation with the blood viscosity.

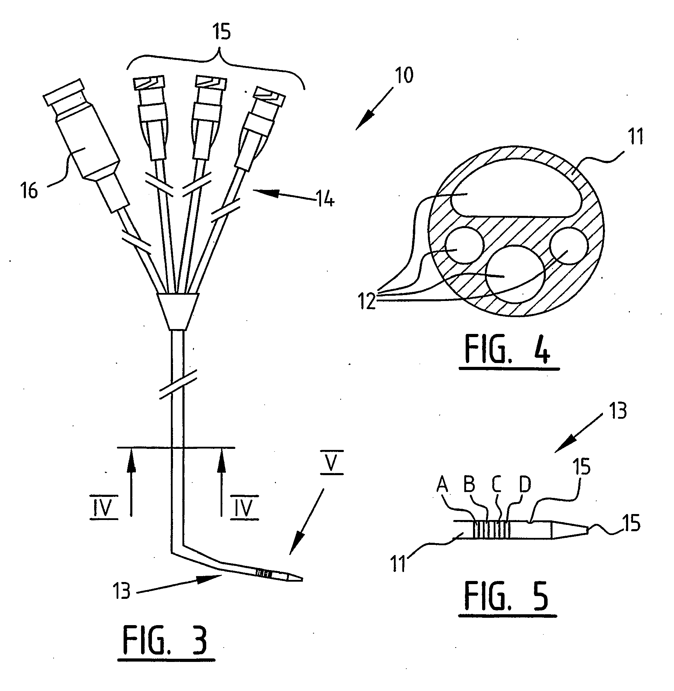

In order to measure the impedance of blood a catheter is preferably used as shown schematically and externally in figures 3-5. Catheter 10 comprises a basic body 11 in which, as figure 4 shows, four lumina 12 are formed in this exemplary embodiment. At proximal end 14 of catheter 10 these lumina are connected to connecting members 15 so that it is possible to supply desired substances via these lumina to the distal end, where they can leave the distal end of the catheter viva openings 15 and be introduced into the bloodstream.

The catheter is formed such that it can be readily positioned with its distal end 13 in the right atrium of the heart.

As shown in more detail in figure 5, distal end 13 of catheter 10 is provided with four electrodes A-D which are each connected to a connector 16 at the proximal end of catheter 10.

Figure 2 shows schematically the device according to the invention with which the impedance of the blood can be measured and the flow in the blood vessel in which the measurement takes place can be calculated.

Shown schematically in figure 2 is catheter 10, comprising the four electrodes A-D and the four connecting conduits leading to connector 16, not specifically shown in figure 2.

These conduits, which extend through basic body 11 of the catheter, are three triaxial conduits 17 and a coaxial conduit 18. In this exemplary embodiment there is further arranged in the distal end of the catheter a thermistor 19 with which a temperature measurement can be carried out.

The device of figure 2 operates as follows.

In this preferred exemplary embodiment five separate frequencies of 20 kHz, 200 kHz, 400 kHz, 600 kHz and 1.2 MHz are successively generated in time in a direct digital synthesizer (DDS) 20. This excitation signal is filtered in filter 21, buffered in 22 and fed to the high-potential electrode A via clamp resistance 23. The low-potential electrode D is connected to earth via a decoupling capacitor (not shown).

In each of the connections connecting electrodes A-D to the electronics a parasitic capacitance of several tens of pF can be measured. Active shielding 24 is therefore used in order to avoid phase and amplification errors. A third earthed shield moreover prevents the emission or entry of undesired signals.

$R_p$ and $C_m$ are calculated in per se known manner from the impedance values at 20, 600 and 1200 kHz.

Via logarithmic amplification detectors 27 and 28 respectively the measuring signal and the excitation signal are fed to a phase detector 29 on the one hand and an amplification detector 30 on the other. A filter 26 is also incorporated in the signal circuit.

The phase signal is supplied via line 33 to AD converter 31 of a microcomputer 32, just as the amplification signal is supplied via line 34 to AD converter 31.

The signal from thermistor 19 is likewise supplied to the AD converter of microcomputer 32 via line 35. A measuring signal supplied via filter 36 and representing the ECG signal is fed via line 37 to AD converter 31.

Microcomputer 32 performs the above stated calculation of the $R_p$ and $C_m$.

$R_p$ has a high correlation with hematocrit and commercially available medical instruments for a direct hematocrit measurement operate according to this method for the purpose of determining this $R_p$.

As noted above, $C_m$ has a high correlation with the blood viscosity.

In order to now be able to determine from the measured $C_m$ the flow volume in the blood vessel in which measurement takes place, the relation between the shear rate, which, as noted above, partly determines the viscosity and thus the $C_m$, and fibrinogen contents is first determined at varying hematocrit.

A suitable approach, as for Newtonian liquids, is to equate the average shear rate in a blood vessel to four times the average flow speed divided by the radius of the blood vessel.

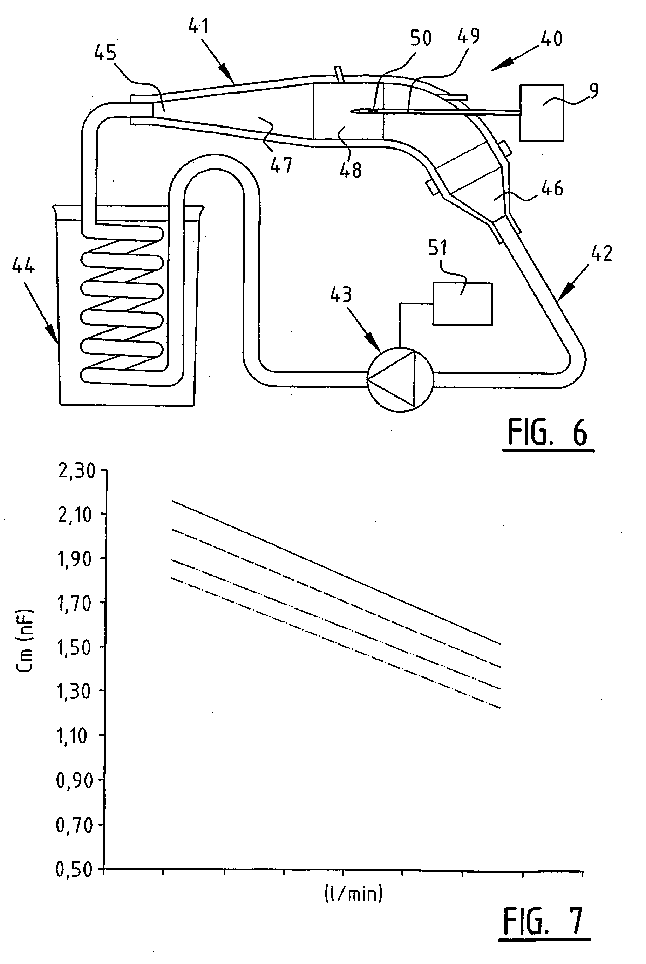

Another possibility is to use a device as for instance shown in figure 6. This device 40 comprises as basic elements a measuring vessel 41 with an inlet 45 and an outlet 46 which are mutually connected via a conduit 42. A pump 43 and a heat exchanger 44 are arranged in this conduit 42.

The results of performed in-vitro measurements have led to the following formulae with which the average flow speed can be determined given the hematocrit, the fibrinogen content and the viscosity (this latter being shown by the $C_m$).

It has been found that there is a close relation between the ohmic resistance and the hematocrit, whereby this formula can also be written as:

4

$$C_m = 0.235 \ exp \ [-3.244 \ flow] + 0.0292 \ fib + 0.0011 \ R_p$$

The current fibrinogen value can be replaced by a constant which equals the average value of fib, which results in the following formula:

$$C_m = 0.224 \ exp \ [-4.035 \ flow] + 0.00146 \ R_p + 0.073$$

These formulae are used in a manner which is further obvious to a skilled person in the field to program the microcomputer so that it can calculate the flow speed from the measured $C_m$ and optionally the entered fibrinogen and hematocrit value $R_p$. Instead of entering the fibrinogen value it is also possible here to make use of the average fibrinogen value, or the measured $R_p$ can be used instead of entering the hematocrit value.

Measuring vessel 41 and conduit 42 are filled with blood. The circulating blood is held at a constant temperature of 37°C in heat exchanger 44.

Measuring vessel 41 is formed such that a uniformly diverging inflow part 47, which runs out into a measuring chamber 48, connects to inlet 45. By choosing the dimensioning of diffusor 47 in appropriate manner in relation to the flow speed of the blood it is possible to ensure in this manner that a laminar flow will occur in measuring chamber 48. In a laminar flow the flow distribution is fully known and the shear rate and flow speed are therefore also known at any point of the cross-section of measuring chamber 48.

The distal end of a catheter 49, which in principle corresponds with the catheter as shown in figure 3, is positioned centrally in measuring chamber 48. Electrodes 50 thereof are connected in the above described manner to a device 9, which corresponds with the device of figure 2.

The blood can circulate in device 40 at a variable speed since pump 43 can be driven at different speeds using a control device 51.

By now measuring the capacitance at differing speeds, relations are found as shown schematically in figure 7. It is indicated that in a significant range of flow speeds there exists an exponentially almost linear relation between the flow speed and the determined $C_m$. This is also apparent from the above stated formulae.

The different conduits shown at different heights in figure 7 indicate that while the linear character of the relation is retained with a varying fibrinogen or hematocrit content, the absolute value varies.

With the viscosity measuring device 40 of figure 6 it is possible to determine in a number of measurements the relation between the flow speed and the $C_m$ of the measured blood at varying hematocrit and fibrinogen contents. From the flow speed, i.e. in this respect the number of litres flowing per minute through device 40, the shear rate at the position of measuring electrode 50 can be determined so that the relation between the average shear rate and the $C_m$ can thus be established at differing fibrinogen and hematocrit values.

When the flow volume must now be determined in a blood vessel, the $C_m$ can be measured in the relevant blood vessel in suitable manner, preferably with catheter 10 and device 9. The hereby found average blood flow speed can be combined with the cross-section of the blood vessel, whereby the flow volume can be calculated.

If it is desired to measure the flow volume of the heart, distal end 13 of catheter 10 can be positioned in suitable manner in the right atrium of the heart.

It will be apparent that the flow speed and therefore the $C_m$ will vary considerably during the heart cycle. The measuring signal is therefore preferably sampled during a determined period in the heart cycle. This period is preferably the end of the systole, the diastole. A gentle flow then occurs in which a good representative measurement can be made. Microcomputer 32 of device 9 can be programmed such that the measuring signal is thus sampled in the desired period of the ECG signal which, as described above, is fed via line 37 to microcomputer 32. The measured and processed impedance signal can be stored in a memory of microcomputer 32 for later processing, or can be processed immediately if the dimensions, in particular the cross-section of the blood vessel in which the measurement takes place, so for instance the right atrium of the heart, are predetermined. This dimension can be suitably determined using echography. This is a per se known technique.

A flow distribution over the cross-section of the blood vessel is further selected. A laminar flow distribution can be chosen in the case of a measurement in the right atrium during the diastole. It has been found that the flow distribution during the diastole in the right atrium can be seen with sufficient accuracy as laminar.

Once the cross-section of the blood vessel has been entered therein and the flow distribution and/or average flow speed has been programmed therein, microcomputer 32 can calculate the flow volume on the basis of the predetermined relation between the shear rate and/or flow speed in the blood and at a determined hematocrit and fibrinogen value, and show it in suitable manner on a display.

In this programmed calculation it is of course taken into account that the measurement has taken place during a determined period of the ECG. On the basis of the ECG and the known heart function a correction factor can be determined for a conversion to the total flow volume during a heart cycle, or it is possible to suffice with the measurement result resulting from the measurement during the determined period of the ECG when at least the greater part of the flow has taken place during this period. It is optionally possible to suffice with the display of a trend of the cardiac output.

According to a further development of the invention, the predetermination of fibrinogen and hematocrit can be dispensed with. Use is made here of a device which corresponds in principle to that of figure 6. A small amount of blood is taken from the person whose flow volume must be measured in a determined blood vessel, for instance the right atrium. This blood is placed in a device such as that of figure 6 and circulated. This device will herein take a small form such that a relatively small quantity of blood can suffice.

The impedance is first measured in the blood vessel in the above described manner. The blood is then circulated in the device according to figure 6 at a speed, to be controlled by the pump, such that the same impedance is measured in the measuring chamber. With the flow speed at which this impedance occurs it is then possible to calculate the flow speed and the flow volume in the blood vessel, wherein the form factors and the like, as indicated above, are taken into consideration.

## Claims

1. Device for determining the flow in a blood vessel, comprising
   means for measuring the electrical impedance in the blood in a cross-section of a blood vessel,
   means for determining the size of the blood vessel cross-section, and
   processing means, comprising
   memory means having stored therein a predetermined relation between the shear rate and the electrical impedance of flowing blood and having stored a theoretical relative flow distribution over the blood vessel cross-section, and
   computing means configured to determine the shear rate from the stored relation and the measured electrical impedance, to determine the average flow speed on the basis of the shear rate and the stored relative flow distribution, and to determine the flow volume from the determined average flow speed and the cross-section.

2. Device as claimed in claim 1, wherein a determined relation between the average flow speed and the electrical impedance of flowing blood is stored in the memory means, and the computing means can determine the average flow speed from the stored relation and the measured electrical impedance and the flow volume from the determined average flow speed and the cross-section.

3. Device as claimed in claim 1 or 2, further comprising a viscosity measuring apparatus comprising a conduit forming a blood vessel flow, moving means incorporated in the conduit for allowing a liquid to flow through the conduit at an adjustable flow speed, electrodes positioned in the conduit and electrical impedance measuring means connected to the electrodes.

## Patentansprüche

1. Vorrichtung zum Bestimmen des Durchflusses in einem Blutgefäß mit
   einem Mittel zum Messen der elektrischen Impedanz in dem Blut in einem Querschnitt eines Blutgefäßes,
   einem Mittel zum Ermitteln der Größe des Querschnitts des Blutgefäßes und
   einem Verarbeitungsmittel mit
   einem Speichermittel, in dem eine vorbestimmte Beziehung zwischen der Schergeschwindigkeit und der elektrischen Impedanz fließenden Blutes gespeichert ist und in dem eine theoretische relative Flussverteilung über den Querschnitt des Blutgefäßes gespeichert ist, und
   einem Berechnungsmittel, das konfiguriert ist zum Ermitteln der Schergeschwindigkeit aus der gespeicherten Beziehung und der gemessenen elektrischen Impedanz, zum Ermitteln der Durchschnittsfließgeschwindigkeit auf der Grundlage der Schergeschwindigkeit und der gespeicherten relativen Flussverteilung, und zum Ermitteln des Durchflussvolumens aus der ermittelten Durchschnittsfließgeschwindigkeit und dem Querschnitt.

2. Vorrichtung gemäß Anspruch 1, bei der
   eine bestimmte Beziehung zwischen der Durchschnittsfließgeschwindigkeit und der elektrischen Impedanz fließenden Blutes in dem Speichermittel gespeichert ist und
das Berechnungsmittel die Durchschnittsfließgeschwindigkeit aus der gespeicherten Beziehung und der gemessenen elektrischen Impedanz und das Durchflussvolumens aus der ermittelten Durchschnittsfließgeschwindigkeit und dem Querschnitt ermitteln kann.

**3.** Vorrichtung gemäß Anspruch 1 oder 2, die weiter eine Viskositätsmessvorrichtung enthält mit
einer Leitung, die einen Blutgefäßfluss bildet,
einem Bewegungsmittel, das in der Leitung eingegliedert ist, um es einer Flüssigkeit zu ermöglichen, mit einstellbarer Fließgeschwindigkeit durch die Leitung zu fließen,
Elektroden, die in der Leitung angeordnet sind, und
einem Mittel zum Messen der elektrischen Impedanz, das mit den Elektroden verbunden ist.

**Revendications**

**1.** Dispositif pour déterminer le flux dans un vaisseau sanguin, comprenant :

des moyens pour mesurer l'impédance électrique dans le sang dans une section transversale d'un vaisseau sanguin,
des moyens pour déterminer la taille de la section transversale du vaisseau sanguin, et
des moyens de traitement, comprenant :
des moyens de mémoire ayant stocké à l'intérieur de ceux-ci une relation prédéterminée entre la rhéofluidification et l'impédance électrique du sang qui circule, et ayant stocké une distribution de circulation relativement théorique sur la section transversale du vaisseau sanguin, et des moyens informatiques configurés pour déterminer la rhéofluidification à partir de la relation stockée et l'impédance électrique mesurée, pour déterminer la vitesse moyenne de circulation en fonction de la rhéofluidification et de la distribution de la circulation relative stockée, et pour déterminer le volume de la circulation à partir de la vitesse moyenne de la circulation déterminée et de la section transversale.

**2.** Dispositif selon la revendication 1, dans lequel une relation déterminée entre la vitesse moyenne de circulation et l'impédance électrique du sang qui s'écoule est stockée dans les moyens de mémoire, et les moyens informatiques peuvent déterminer la vitesse moyenne de circulation à partir de la relation stockée et de l'impédance électrique mesurée et le volume d'écoulement à partir de la vitesse moyenne de circulation déterminée et de la section transversale.

**3.** Dispositif selon la revendication 1 ou 2, comprenant en outre un appareil de mesure de viscosité comprenant un conduit formant un flux de vaisseau sanguin, des moyens de déplacement incorporés dans le conduit pour permettre à un liquide de s'écouler à travers le conduit à une vitesse d'écoulement ajustable, des électrodes positionnées dans le conduit et des moyens de mesure d'impédance électrique raccordés aux électrodes.

## FIG. 1

## FIG. 2

15

10

16

14

11

12

FIG. 4

13

15

A B C D

15

11

FIG. 5

IV IV V

13

FIG. 3

EP 1 933 701 B1

FIG. 6

FIG. 7

10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004075928 A2